(19) Europäisches Patentamt
European Patent Office
Office européen des brevets

(11) EP 2 789 685 B1

(12) EUROPEAN PATENT SPECIFICATION

(45) Date of publication and mention
of the grant of the patent:
01.08.2018 Bulletin 2018/31

(51) Int Cl.:
C12N 5/00 (2006.01)

(21) Application number: 13001816.1

(22) Date of filing: 09.04.2013

(54) **3D CELL CULTURE SUBSTRATE COMPRISING POLYMER BRUSHES**

3D-ZELLENKULTURSUBSTRAT MIT POLYMERBÜRSTEN

SUBSTRAT DE CULTURE CELLULAIRE EN 3D COMPRENANT DES BROSSES DE POLYMÈRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.10.2014 Bulletin 2014/42**

(73) Proprietors:
• **Leibniz-Institut für Polymerforschung Dresden e.V.**
**01069 Dresden (DE)**
• **Technische Universität Dresden**
**01062 Dresden (DE)**

(72) Inventors:
• **KÖNIG, Ulla**
**01219 Dresden (DE)**
• **UEDA, Yuichiro**
**01219 Dresden (DE)**
• **UHLMANN, Petra**
**01326 Dresden (DE)**
• **HANKE, Thomas**
**13187 Berlin (DE)**

(74) Representative: **Andrae | Westendorp Patentanwälte Partnerschaft Uhlandstraße 2 80336 München (DE)**

(56) References cited:
• **SINA BURKERT ET AL: "Protein Resistance of PNIPAAm Brushes: Application to Switchable Protein Adsorption", LANGMUIR, vol. 26, no. 3, 2 February 2010 (2010-02-02), pages 1786-1795, XP055068086, ISSN: 0743-7463, DOI: 10.1021/la902505q**

• **NAOKAZU IDOTA ET AL: "Novel temperature-responsive polymer brushes with carbohydrate residues facilitate selective adhesion and collection of hepatocytes", SCIENCE AND TECHNOLOGY OF ADVANCED MATERIALS, vol. 13, no. 6, 1 December 2012 (2012-12-01), page 064206, XP055068103, ISSN: 1468-6996, DOI: 10.1088/1468-6996/13/6/064206**

• **MIZUTANI ET AL: "Preparation of thermoresponsive polymer brush surfaces and their interaction with cells", BIOMATERIALS, ELSEVIER SCIENCE PUBLISHERS BV., BARKING, GB, vol. 29, no. 13, 7 February 2008 (2008-02-07), pages 2073-2081, XP022513862, ISSN: 0142-9612, DOI: 10.1016/J.BIOMATERIALS.2008.01.004**

• **QIAN YU ET AL: "Protein Adsorption and Cell Adhesion/Detachment Behavior on Dual-Responsive Silicon Surfaces Modified with Poly( N -isopropylacrylamide)- block -polystyrene Copolymer", LANGMUIR, vol. 26, no. 11, 1 June 2010 (2010-06-01), pages 8582-8588, XP055068368, ISSN: 0743-7463, DOI: 10.1021/la904663m**

• **LEE JUNGWOO ET AL: "Three-dimensional cell culture matrices: state of the art", TISSUE ENGINEERING PART B-REVIEWS, MARY ANN LIEBERT, INC. PUBLISHERS, US, vol. 14, no. 1, 10 March 2008 (2008-03-10) , pages 61-86, XP002603255, ISSN: 1937-3368, DOI: 10.1089/TEB.2007.0150 [retrieved on 2008-03-02]**

• **Eelco Fennema ET AL: "Spheroid culture as a tool for creating 3D complex tissues", Trends in Biotechnology, vol. 31, no. 2, 1 February 2013 (2013-02-01), pages 108-115, XP055086215, ISSN: 0167-7799, DOI: 10.1016/j.tibtech.2012.12.003**

Remarks:
    The file contains technical information submitted after
    the application was filed and not included in this
    specification

Remarks:
    The file contains technical information submitted after
    the application was filed and not included in this
    specification

## Description

### Technical field

The present invention relates to the use of a 3D cell culture substrate for the culture of 3D cell spheroids, and a method for culturing 3D cell spheroids.

### Background of the Invention

Methods currently used for culturing cells especially in two dimensions (2D) possess limitations. The culture of cells in two dimensions does not reproduce the human or animal physiology and anatomy, adequately. Creating a third dimension (3D) for cell culture is obviously more relevant and shows the cell biological microenvironment more realistically because 3D cultures more closely reflect the environment experienced by normal cells in the body which are completely surrounded by other cells, membranes, fibrous layers and adhesion proteins.

The application of 3D cell culture techniques has received increasing attention due to results showing significant differences between the cellular phenotype and biological response of cells cultured in monolayer (2D) in comparison with 3D cell culture. It is known that 3D methods facilitate greater cell-cell contacts and interactions of cells with the extracellular matrix (ECM), allowing cells to adapt to their native morphology which may influence signalling activity, gene and protein expression and drug metabolism. 3D culture systems often promote the levels of cell differentiation and tissue organization.

Approaches providing a 3D environment for cell cultures have been successfully employed especially on drug discovery studies, tumor biology, tissue engineering, and stem cell research where the cellular microenvironment is known to have a profound influence on the biology of the stem cells within it (Yoshii Y et al., Biomaterials. 2011 Sep;32(26):6052-8.).

The primary objectives for developing 3D cell culture systems vary widely and range from engineering tissues for clinical delivery as well as the development of 3D cell culture models for drug screening through to 3D printing and 3D culture-on-chips. When entering the third dimension it is often necessary to consider the design of scaffolds or substrates supporting the arrangement of cells. The biological cross talk between cells and scaffold materials is controlled by characteristics like surface properties or biocompatibility.

Certainly, even scaffold-free methods need to use particular materials and equipped support. For example, the methylcellulose culture system mostly carried out in U-shape-non-stick-wells is not able to fabricate pure cell aggregates because of the contamination with methylcellulose inside of the formed spheroids. This technique is time-consuming and the practical procedure has to be adjusted to each kind of cells.

Using Polyurethane foam as another matrix is a further unique technique suitable for fabricating 3D cell constructs but to harvest spheroids from this foam shows up practical difficulties.

Furthermore, a number of studies have previously introduced methods of generating 3D cell spheroids, including the hanging drop, spinner flask, and micro-chamber methods. The hanging drop method is popular because it is a one-step-procedure whereby the number of the cells can be controlled. It is even suitable and available as automation system (Perfecta3D™). Under simulated microgravity environment cells can form 3D cell spheroids by using specific equipments such as Rotating Wall Vessel, Roller Tube, Spinner Flask, and Gyratory Shaker.

Indeed, all of these methods have certain disadvantages and difficulties in common. Most of them are not able to control or adjust the size and the desired morphology of the 3D cell spheroids, the growth rate of the spheroids is low and takes few days and the optimization of the specific equipments for each cell type is complicated and time-consuming (Kim TG et al., Adv Funct Mater. 2010;20(14):2303-9.).

Furthermore, if the formed spheroids have an irregular size and a differing number of containing cells this could cause problems for further experiments and the cell-cell interaction could be not enough to take an advantage of 3D spheroid culture systems.

Burkert et al: "Protein Resistance of PNIPAAm Brushes: Application to Switchable Protein Adsorption", LANGMUIR, vol. 26, no. 3, 2 February 2010, pages 1786-1795 discloses switchable polymer brushes (PNIPAAm, P2VP homopolymers or binary polymers) and their temperature-dependent adsorption behavior.

Idota et al: "Novel temperature-responsive polymer brushes with carbohydrate residues facilitate selective adhesion and collection of hepatocytes", SCIENCE AND TECHNOLOGY OF ADVANCED MATERIALS, vol. 13, no. 6, 1 December 2012, page 64206 discloses the preparation of random and block copolymer brushes comprising PNIPAAM and LAMA (2-lactobionamidoethyl methacrylate) to investigate the effects of grafting architectures of these copolymers on adhesion and collection of hepatocytes and demonstrates selective adhesion or detachment of single cells.

Mizutani et al: "Preparation of thermoresponsive polymer brush surfaces and their interaction with cells", BIOMATERIALS, vol. 29, no. 13, 7 February 2008, pages 2073-2081 discloses the preparation of PNIPAAM homopolymer brush surfaces on polystyrene substrates to be used as thermoresponsive substrates for the preparation of cell sheets from attachment-dependent cells having strong adhesive properties.

Qian Yu et al: "Protein Adsorption and Cell Adhesion/Detachment Behavior on Dual-Responsive Silicon Surfaces Modified with Poly(N-isopropylacrylamide)-block-polystyrene Copolymer", LANGMUIR, vol. 26, no. 11, 1 June 2010, pages 8582-8588 discloses the preparation of substrates having poly(N-isopropylacrylamide)-block-polystyrene (PNIPAAM-b-PS) brushes to study the stimuli-responsive properties of PNIPAAM-b-PS-modified surfaces. Qian Yu et al. shows that the cell culture surfaces disclosed therein comprising PNIPAAM either prevents cell attachment and growth or may lead to cell adhesion in a spindle-like shape with cells having pseudopods and cell processes.

It is therefore an object of the present invention to provide the use of a 3D cell culture substrate for the culture of 3D cell spheroids which allows the simple and rapid preparation of 3D cell spheroids with controlled size and morphology which are free of gel-matrix or scaffold material and can easily be obtained from the substrate.

It is another object of the present invention to provide a method for culturing 3D cell spheroids.

## Summary of the Invention

According to one aspect of this invention, the use of a 3D cell culture substrate comprising polymer brushes, wherein the polymer brushes comprise poly (N-isopropylacrylamide) polymers for the culture of 3D cell spheroids, wherein the polymer brushes comprise or consist of binary polystyrene-poly(N-isopropylacrylamide) brushes with a ratio of PNIPAAm of at least 80%, and wherein the grafting density of the polymer brushes is between 0.01 and 0.2 nm$^{-2}$ is provided.

Preferably, the polymer brushes comprise polymers that are water-soluble and uncharged.

Preferably the polymer brushes are prepared by a grafting to process.

Preferably, the grafting density of the polymer brushes is between 0.02 and 0.15 nm$^{-2}$.

Preferably, the polymer brushes have a polydispersity index (PDI) of at most 1.5, preferably at most 1.4.

According to one aspect of the invention, the cells are human cells, more preferably Normal Human Dermal Fibroblasts (NHDF) or human Mesenchymal Stem Cells (hMSC).

Preferably, the cells are seeded in a concentration of less than 4x10$^5$ cells/ml, preferably in a concentration of between 1x10$^s$ cells/ml to 2x10$^5$ cells/ml.

A second aspect of the present invention provides a method for culturing 3D cell spheroids on a cell culture substrate comprising polymer brushes as described and claimed herein.

Preferably, the cells are seeded in a concentration of less than 4x10$^5$ cells/ml, preferably in a concentration of between 1x10$^s$ cells/ml to 2x10$^5$ cells/ml.

According to one embodiment, the 3D cell spheroids are released from the substrate by cooling to a temperature of about 30°C or below.

Preferably, the 3D culture substrates and/or spheroids cultured according to the claimed method do not contain any scaffold or gel-matrix material.

According to one embodiment of the various aspects of the invention, the polymer brushes do not contain RGD peptides, preferably also not other peptides.

## Detailed Description of the Invention

As known to the skilled person, 3D cell culture substrates are principally different from 2D cell culture substrates. According to one aspect of the present invention, the 3D culture substrate comprises polymer brushes. It has been found that 3D culture substrates should preferably comprise brushes with water soluble and uncharged polymers. According to one aspect, the polymer brushes comprise poly(N-isopropylacrylamide) polymers.

It has been found that advantageous substrates are obtained if the polymer brushes are obtained by a grafting to process. This allows a highly ordered and uniform structure of the polymer brushes offering advantages for the 3D substrate.

Preferably, in the 3D cell culture substrate the polymer brushes have a polydispersity index (PDI) of at most 1.5, preferably at most 1.4.

The polydispersity index (PDI) is a measure of the distribution of molecular mass in a given polymer sample. The PDI calculated is the weight average molecular weight ($M_w$) divided by the number average molecular weight ($M_n$). It indicates the distribution of individual molecular masses in a batch of polymers. The PDI has a value equal to or greater than 1, but as the polymer chains approach uniform chain length, the PDI approaches unity. The PDI from polymerization is often denoted as:

$$PDI = M_w/M_{n,}$$

where $M_w$ is the weight average molecular weight and $M_n$ is the number average molecular weight. $M_n$ is more sensitive to molecules of low molecular mass, while $M_w$ is more sensitive to molecules of high molecular mass.

The $M_n$ of the PNIPAAm polymers is preferably in the range of about 20000 to 400000, more preferably from 30000 to 300000.

The grafting density of the polymer brushes is preferably between 0.02 and 0.15 nm-$^2$.

According to a reference aspect of the invention, the polymer brushes consist of poly(N-isopropylacrylamide) homopolymer brushes.

According to the present invention, the polymer brushes comprise or consist of binary polystyrene-poly(N-isopropylacrylamide) brushes with a ratio of PNIPAAm of at least 80%.

It has been found that particularly suitable 3D cell culture substrates are obtained if polymer brushes have a ratio of PNIPAAm of at least about 80%. In particular, the ratio is expressed as the percentage of PNIPAAm units in the polymer brush on the basis of the overall number of units in the polymer brush. Thus, the polymer brushes have a ratio (content) of PNIPAAm of at least about 80%.

The present invention concerns the use of the substrate as described herein for the culture of 3D cell spheroids. I has been surprisingly found that these substrates have advantageous properties, including (limited) cell adhesion properties which allow the culture of 3D culture as compared to 2D monolayers adhering to the substrate.

According to one embodiment of the invention, the cultured cells are human cells or cell lines such as Normal Human Dermal Fibroblasts (NHDF) or human Mesenchymal Stem Cells (hMSC).

It has been surprisingly found that 3D cell culture is beneficially influenced if the cells are seeded in a concentration of less than $4 \times 10^5$ cells/ml, preferably in a concentration of between $1 \times 10^5$ cells/ml to $2 \times 10^5$ cells/ml. This helps to increase the speed of formation and uniformity of the 3D cell spheroids.

According to one embodiment of the invention, the 3D cell spheroids have a size of 50 to 500 $\mu$m, preferably 50 to 200 $\mu$m, in particular when attached to the substrate.

Another aspect of the present invention concerns a method for culturing 3D cell spheroids on a cell culture substrate as defined herein and comprising polymer brushes as defined herein.

According to one embodiment of the invention, the 3D cell spheroids are released from the substrate by cooling to a temperature of about 30°C or below. In some cases, the 3D cell spheroids may also detach from the substrate without a stimulus when reaching a certain size upon continuing the culture for a time period.

According to one embodiment of the invention, the spheroids preferably do not contain any scaffold or gel-matrix material. This is a particularly preferred advantage provided by the present invention.

According to one embodiment of the present invention, the 3D substrate is not sterilized after preparation of the polymer brushes. Thus, one advantage of the present invention is that sterilization may be achieved by the process of the formation of the polymer brushes (and thus the substrate) themselves. If subsequent sterilization is required, it is preferable not to use gamma radiation.

By combining the potential of the modern nanotechnology with the current knowledge and understanding in the fields of biotechnology, regenerative medicine and tissue engineering, the inventors recognized the chance to create smart and functional high-tech materials which are able to adapt to their environment and employ these materials for the growing field of 3D cell culture.

The ability to tailor the morphological and chemical structure of these materials provides the opportunity of a distinguished control of material properties. Many of the smart, intelligent materials have surfaces that dynamically alter their physicochemical properties in response to changes in their environmental conditions such as temperature, ionic strength, solvent polarity, electric/magnetic field, light or small (bio-)molecules. They are capable to manipulate their interfacial properties at the solid/water interface and are able to respond to both internal and external environmental stimuli, reversibly (Ma PX, Adv Drug Deliv Rev. 2008 Jan 14;60(2): 184-98).

Polymers, more than any other material, offer this possibility to fine-tune their thermal, optical and electrical properties and bring up this interest which has expanded into the broadly practical realm of functional surfaces. Specifically, polymer brushes have recently been identified as smart nanoscale and stimuli-responsive materials which can achieve these requirements.

Polymer brushes are polymer coatings in which polymer chains are tethered on one end to a surface at sufficiently high grafting density. The degree to which the chains are stretched away from the surface is determined by competition between the entropic energy associated with chain stretching and the excluded volume interactions between different segments of the brush. Polymer brushes are typically prepared through physisorption or covalent attachment. Covalent attachment can be achieved using "grafting-to" and "grafting-from" techniques.

"Grafting to" means the tethering of preformed functionalized polymer chains to a surface. The "grafting from" approach uses a surface immobilized initiator layer and subsequent in situ polymerization to generate the polymer brush.

Polymer brushes are well-defined and surface-confined macromolecular architectures that offer performance gains and benefits in the areas of biotechnology as well as nanotechnology. Currently, one of the most promising technical advances and contributions in using polymer brushes have been associated with their biointerfacial applications, as defined by the intersection of these brushes within the biological environment including the interaction with biological macromolecules, supra-molecular assemblies, and cells.

In particular, several relevant studies have recognized that polymers like poly(ethylene glycol) or thermo-responsive poly(N-isopropylacrylamide) are regarded as appropriate candidates for such biomedical application (Burkert S et al., Langmuir. 2010 Feb 2;26(3):1786-95. and Bittrich E et al., Langmuir. 2012 Feb 21;28(7):3439-48.).

However, such polymers have previously only been considered in the context of standard 2D cell culture or production of cell sheets (Nagase K et al, J R Soc Interface. 2009 June 6; 6(supp3): S293-S309.).

The present inventors surprisingly discovered that polymer brushes which comprise polymers that are uncharged and water-soluble are relatively resistant against the adsorption of proteins from the cell culture media. High adsorption of unspecific protein significantly increases the adhesion of cells and ultimately leads to the formation of cell layers covering the surface of the polymer brushes.

The present invention is based on the recognition that polymer brushes may be designed in a way that unspecific protein adsorption is minimized leading to a well-defined pattern of cell adhesion which allows the formation of 3D cell spheroids. This effect of protein resistance may be caused by a very low dispersity of the polymer chains grafted to the substrate. According to one hypothesis, the highly homogeneous assembly of the water-soluble and uncharged polymer chains could enable the formation of highly ordered water structures which is energetically favoured in the absence of adsorbed proteins.

The inventors have surprisingly found that the 3D culture substrates, especially those comprising poly(N-isopropylacrylamide) provide a surface which is especially suitable for 3D sheroid culture.

The term "culture" is used herein to include the growing of 3D cell spheroids from seeding cells or cell dispersions.

An advantage of the present invention is that it allows the formation of 3D cell spheroids composed exclusively of cells and containing no scaffold material which might be a valuable biologic material for tissue engineering applications.

A method for culturing 3D cell spheroids according to the present invention could be the simplest method to achieve more effective cell-cell interactions within *in vitro* systems.

The substrate according to the present invention has the further advantage that it can easily be produced by grafting the respective polymer brush coatings on conventional 2D cell culture equipments such as flasks and well plates and therefore can be handled easily. The thin polymer brush films on nanoscale-level have great optical properties (comparable to glass) for high performance imaging.

The 3D culture substrate according to the present invention allows fabricating 3D cell spheroids within a time-saving one-step-procedure eliminating extra material contaminations because it is scaffold-free and gel-matrix-free. The number of cells inside of the spheroids can be controlled by the polymer grafting process generating a patterned surface with different area size of polymer brushes which are important for the initial attachment in order to limit the number of cells.

The harvest of the 3D cell spheroids is easy from the substrate according to the present invention because a temperature responsive polymer is used, and the temperature-sensitivity below the LCST of the polymer (in case of poly(N-isopropylacrylamide): ~32°C) may be employed to detach spheroids from the polymer brush surface.

Other stimulus-responsive polymers responding to stimuli including temperature, pH, and ion concentration may be used. Using the respective stimulus, the polymer brush surface can be repeatedly reused for re-culturing spheroids which are gain to be reproducible and uniform. The present invention may also be used for co-culturing in three dimensions without any dramatically change of current employed protocols whereby spheroids on polymer brush coatings preserve differentiation characteristics and are amenable for high throughput screening.

A promising advantage of the present invention is the possible control the nano-scale level of the surface by current technology. It is now widely recognized that there are many advantages of 3D culture methods, however to develop a biomimetic microenvironment such as in the present invention requires a multidisciplinary approach and expertise.

The following excerpt of search results are given to further illustrate certain aspects of the invention. They shall not be construes to be limiting or in contradiction to the embodiments of the invention described above.

The figures show:

**Fig. 1:**
Zeta potential measurements of PNIPAAm with different molecular weights

**Fig. 2:**
Swelling behavior of homopolymer brushes, comparison of the dry and the swollen layer thickness at room temperature

**Fig. 3:**
the percentage amount of buffer solution within the different brush layers

**Fig. 4:**
LCST behavior of PNIPAAm (94kDa); change of layer thickness in dependence on temperature

**Fig. 5:**
Influence of sterilisation conditions on layer thickness and water contact angle of homopolymer brushes

**Fig. 6:**
Unspecific Protein Adsorption with Serum (FCS) onto homopolymer brushes, comparison to PS well plate as control

**Fig. 7a:**
NHDF after 72 h Cell Culture on Polymer Brushes,
Staining Method: DAPI (Nucleus)/Phalloidin AlexaFlour48 (FActin)

    (**A**) PS (**B**) P2VP (**C**) PAA (**D**) PNIPAAm (94 kDa)

**Fig. 7b:**
hMSC after 72 h Cell Culture on

    (**A**) PS Well Control, Polymer Brushes: (**B**) PS (**C**) P2VP (**D**) PAA (**E**) PNIPAAm (94 kDa)

**Fig. 8:**
Viability Test (UptiBlue, 3 h) after 72h adhesion on polymer brush samples

**Fig. 9:**
Viability Test (UptiBlue, 3 h) after 72h adhesion on different sterilized polymer brush samples,

**Fig. 10:**
hMSC after72 h Cell Culture on PNIPAAm with/without UV Sterilization and with/without 10%FCS in Culture Medium

    (**A**) UV(+) FCS(+), (**B**) UV(+) PS Well Control, (**C**) UV(+) FCS(-)
    (**D**) UV(-) FCS(+), (**E**) FCS(-) PS Well Control, (**F**) UV(-) FCS(-)

**Fig. 11:**
Compariosn of NHDF and hMSC on PNIPAAm Polymer Brushes after 72 h Cell Culture Staining Method: Hoechst33342 (Nucleus) with LSM and DIC Image

    (**A**) PAA Polymer Brushes, NHDF Monolayer
    (**B**) PNIPAAm 94 kDa, NHDF Spheroid
    (**C**) PAA Polymer Brushes, hMSC Monolayer
    (**D**) PNIPAAm 94 kDa, hMSC Spheroid

**Fig.12:**
Comparison of the two viability tests: LDH and UptiBlue assay; NHDF adhesion 72 h on each Polymer Brushes Samples

**Fig. 13:**
Spheroid Re-seeding Experiment (Transferred into new PS Culture Well)

    (A) NHDF Spheroid (PNIPAAm 94 kDa), (B) After 72 h transferred NHDF Spheroid
    (C) hMSC Spheroid (PNIPAAm 94 kDa), (D) After 72 h transferred hMSC Spheroid

**Fig.14:**
Different Cell Densities of NHDF on PNIPAAm Polymer Brushes after 72 h Cell Culture

    (**A**) Low (2.5 x 10^4)
    (**B**) Mid (5.0 x 10^4, Standard Protocol for this study)
    (**C**) High (1.0 x 10^5)
    (**D**) Mid + 24 h Pre-incubation with culture medium (5.0 x 10^4)

**Fig. 15:**
viability test (UptiBlue 3h) of NHDF after 72h cultivation:
Low (2.5x10^4), Mid (5.0x10^4) High Density(1x10^5) and Mid+24 h Medium Pre-incubation

**Fig. 16:**
viability test (UptiBlue 3 h) of hMSC, adhered 72h on Polymer Brushes: UV(+) and UV(-), FCS(+) and FCS(-)

**Fig. 17:**
hMSC on PNIPAAm polymer brushes (94 kDa), after 5days cell culture under serum free conditions

**Fig. 18a:**
NHDF after 72 h cell culture on PNIPAAm polymer brushes

(**A**) 45 kDa, (**B**) 56 kDa , (**C**) 94 kDa
(**D**) 91 kDa + Hydrophobic End, (**E**) 135 kDa + Hydrophobic End, (**F**) 178 kDa + Hydrophobic End

**Fig. 18b:**
hMSC after 72 h cell culture on PNIPAAm polymer brushes

(**A**) 45 kDa, (**B**) 56 kDa , (**C**) 94 kDa
(**D**) 91 kDa + Hydrophobic End, (**E**) 135 kDa + Hydrophobic End, (**F**) 178 kDa + Hydrophobic End

**Fig. 19a:**
NHDF after 72 h Cell Culture on Binary Polymer Brushes

(**A**) PS/PNIPAAm (94 kDa) 80:20, (**B**) PS/PNIPAAm (94 kDa) 20:80
(**C**) PAA/PNIPAAm (94 kDa) 80:20, (**D**) PAA/PNIPAAm (94 kDa) 20:80

**Fig. 19b:**
hMSC after 72 h Cell Culture on Binary Polymer Brushes

(**A**) PS/PNIPAAm (94 kDa) 80:20, (**B**) PS/PNIPAAm (94 kDa) 20:80
(**C**) PAA/PNIPAAm (94 kDa) 80:20, (**D**) PAA/PNIPAAm (94 kDa) 20:80

**Fig. 20:**
hMSC on Binary Polymer Brushes after 72 h Cell Culture

(A) PAA-PNIPAAm (20:80), (B) After Cooling Process (4°C for 10 min)
(C) PS-PNIPAAm (20:80), (D) After Cooling Process (4°C for 10 min)

**Fig. 21:**
Reused PNIPAAm Polymer Brushes:

(**A**) After 72 h with NHDF (Spheroid, PNIPAAm 94 kDa
(**B**) After Trypsin/EDTA Treatment (72 h at 37°C)
(**C**) After 72 h Reuse, Spheroids were formed again (PNIPAAm 94 kDa)

The driving power of all carried out experiments was the idea of creation a 3D cell culture method reflecting the natural cell-cell environment inclusive their cross-talks with optimised adjusted cell culture conditions on adapted minimised surfaces.

Characterisation of applied polymer brushes

End-grafted nano-scaled polymer brush coatings are promising for the creation of responsive surfaces. The applied surface characterisation methods allows the evaluation of the physico-chemical interface properties of the polymer brushes and thus the estimation of the quality of grafted polymer brush layers. Here typically confirmed properties of homopolymer brushes under dry and therefore deswelling conditions were summarised in Table A. The consequent keep of the parameters of the brush preparation manual results in reliable and reproducible data.

TABLE A Characteristics of prepared homopolymer brushes:

| HOMOPOLYMER **BRUSH** | *dry layer thickness* **d** [nm] | **grafting density** $\sigma$ [nm$^{-2}$] | **advancing contact angle** $\theta_A$ [°] at 22°C |
|---|---|---|---|
| PS | 16.5 ± 0.5 | 0.201 | 88.9 ± 1.1 |
| **P2VP** | 7.9 ± 0.2 | 0.120 | 73.5 ±1.5 |
| **PAA (hydrolysed)** | 5.7 ± 0.2 | 0.159 | 30.5 ± 1.8 |
| **PNIPAAm** | | | |
| **(45kDa)** | 8.9 ± 0.3 | 0.110 | 71.5 ± 2.1 |
| **(56kDa)** | 10.2 ± 0.5 | 0.070 | 69.7 ± 1.4 |
| **(94kDa)** | 9.5 ± 0.2 | 0.084 | 70.9 ± 1.9 |
| **PNIPAAm$^{C12*}$** | | | |
| **(91kDa)** | 6.5 ± 0.2 | 0.063 | 70 |
| **(135kDa)** | 9.0 ± 0.2 | 0.054 | 84 |
| **(178kDa)** | 8.5 ± 0.2 | 0.038 | 78 |
| **\* included hydrophobic carbohydrate chain (RAFT-C12-end group)** | | | |

In this evaluation we focus on PNIPAAm brushes because of their stimuli-responsive characteristics which could be essential for further controlled cell adhesion experiments. As can be seen in Table B, the water contact angle measurements have been monitoring the temperature sensitivity of variable PNIPAAm polymer brushes.

TABLE B Advancing contact angle values ($\theta$A) of PNIPAAm homopolymer and binary brushes at 22°C (room temperature) and at 40°C; binary polymer brushes were prepared with PNIPAAM 45kDa, dry layer thickness (d) in nm

| HOMOPOLYMER **BRUSH** | $\theta_A$ [°] at 22°C | $\theta_A$ [°] at 40°C | **BINARY POLYMER BRUSH** | | $\theta_A$ [°] at 22°C | $\theta_A$ [°] at 40°C |
|---|---|---|---|---|---|---|
| **PNIPAAm** | | | **PNIPAAm-PAA** (d = 7.3nm) | **20:80** | 60 | 41 |
| **(45kDa)** | 72 | 90 | | | | |
| **(56kDa)** | 70 | 89 | **PNIPAAm-PAA** (d = 6.9 nm) | **80:20** | 74 | 71 |
| **(94kDa)** | 71 | 87 | | | | |
| **PNIPAAm$^{C12*}$** | | | **PNIPAAm-PS** (d = 10.7 nm) | **20:80** | 87 | 82 |
| **(91kDa)** | 70 | 97 | | | | |
| **(135kDa)** | 84 | 96 | **PNIPAAm-PS** (d = 9.2 nm) | **80:20** | 85 | 80 |
| **(178kDa)** | 78 | 95 | | | | |

It is known that the dry layer thickness and the grafting density influence the advancing contact angle. Therefore in our experiments where these parameters are not constantly only the observed tendency will be discussed. Similar changes toward a more hydrophobic behaviour above the LCST like in the literature were monitored for the PNIPAAm homopolymer brush surfaces. Additionally known is a so-called stick-slip behaviour especially observed on PNIPAAm 45kDa brush surfaces during dynamic contact angle measurements. The stick slip pattern could manipulate the wettability of binary PNIPAAm brush surfaces in particular.

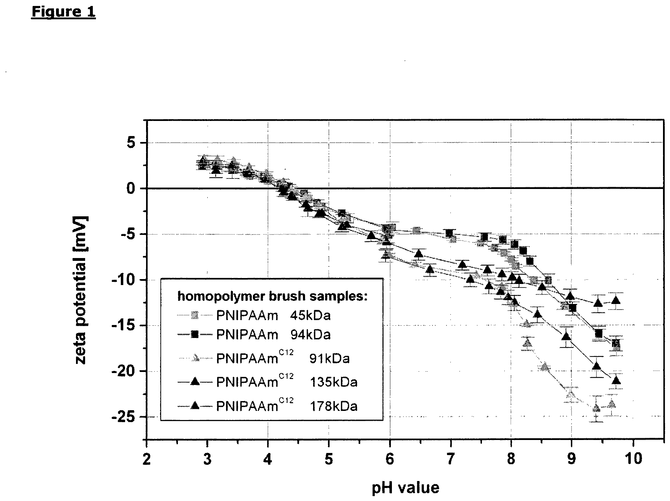

Zeta-potentials were calculated from pH sensitive streaming potential measurements (Fig. 1). Hereby the zeta-potential is negative for all PNIPAAm homopolymer brushes in the investigated pH regime and their isoelectric points were in the range of pH 4.5. The trends are similar of all brushes and the presence of negative surface charges can be concluded. The charges are due to the preferential adsorption of negative ions (e.g. chloride, phosphate) on the surface. Varying the molecular weight of PNIPAAm does not lead to significant changes in zeta-potential.

PNIPAAm is one of the best investigated water soluble polymers and undergoes a phase transition in aqueous solutions at its LCST of 32°C. Certain with the phase transition associated effects are the increase of the hydrophobicity of the brush surface above the LCST, the collapse of the polymer chains and changes in the brush density profile. Among with the contact angle investigations in situ spectroscopic ellipsometric swelling measurements supported the detection of the mentioned temperature sensitivity of PNIPAAm.

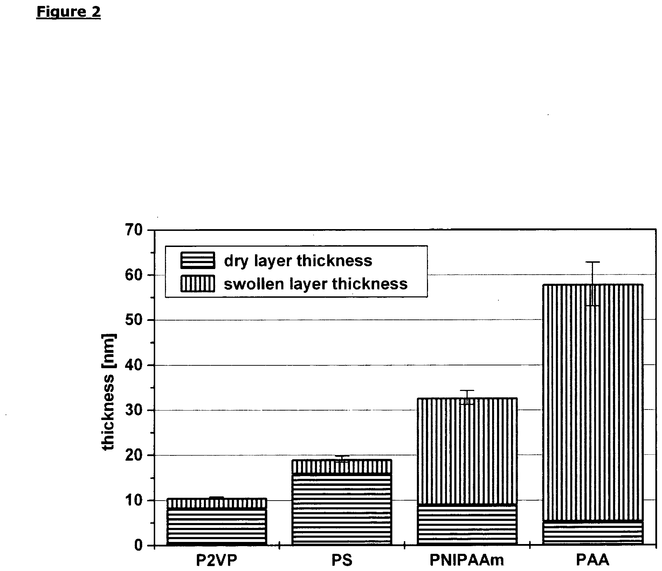

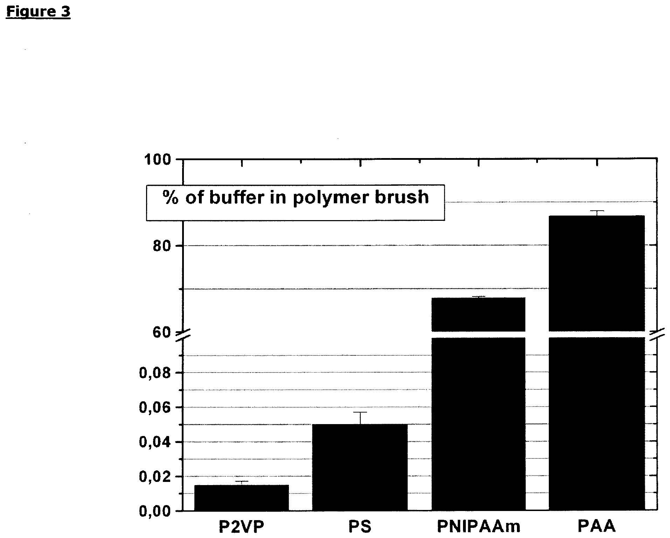

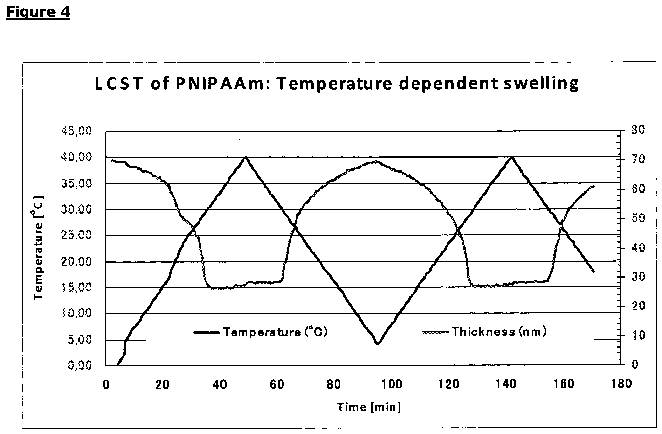

Generally, figures 2 and 3 show the pronounced swelling behaviour of PNIPAAm and PAA homopolymer brushes compared to the PS and P2VP brushes by displaying the fitted swollen layer thickness and the percentage amount of absorbed buffer solution within the brush layers. To complete the swelling experiments the results of temperature dependent swelling in buffer solution with fixed physiological pH at 7.4 are monitored in figure 4 for PNIPAAm homopolymer brush. Swelling was detected in PBS buffer solution in a heating and cooling cycle. A characteristically increase in swollen PNIPAAm brush thickness could be estimated accomplished with a decrease in the refractive index (the latest data not shown). Also the swelling behaviour is dependent on the grafting density.

Thus the continuing approach needs more attention to precisely prepared polymer brush layers focused on the grafting density.

Impact of Sterilization

Currently used sterilization techniques such as ethylene oxide, γ-irradiation, and steam sterilization could introduce undesired consequences, especially in polymeric materials.

Therefore this study investigates the impact of different sterilization processes on structural integrity and stability of homopolymer brush coatings. Both selected sterilization methods (y-irradiation and UV) altered the surface properties of the homopolymer brush grafted samples in terms of surface composition and wettability as seen in table C.

TABLE C XPS evaluation: Elementary composition of homopolymer brush surfaces before (non) and after sterilization process (UV60min, $\gamma$-irradiation)

| | PS | | | P2VP | | | PNIPAAm | | | PAA | | |
|---|---|---|---|---|---|---|---|---|---|---|---|---|
| | **non** | **UV60** | $\gamma$ | **non** | **UV60** | $\gamma$ | **non** | **UV60** | $\gamma$ | **non** | **UV60** | $\gamma$ |
| **[N] : [C]** | - | - | - | 0.115 | 0.129 | 0.129 | 0.155 | 0.156 | 0.160 | 0.008 | 0.005 | 0.006 |
| **[O] : [C]** | 0.036 | 0.002 | 0.021 | 0.101 | 0.049 | 0.208 | 0.246 | 0.157 | 0.179 | 0.452 | 0.553 | 0.592 |
| **[Si] : [C]** | 0.018 | 0.004 | 0.003 | 0.090 | 0.031 | 0.063 | 0.107 | 0.031 | 0.046 | 0.075 | 0.087 | 0.148 |

In order to discuss the influence of sterilisation on the homopolymer brush surfaces the C1s spectra were analysed (spectra not separately illustrated). It is possible to detect changes of brush surface composition due to functionalization reactions after sample treatment. Firstly, the PS C 1s spectrum has not been changed significantly after each sterilization method. However the oxygen content of the gamma-irradiated surface was slightly increased compared to UV-treatment. Therefore 2 further component peaks appeared related to oxidised carbon species (C-O compound of ether or alcohol) or to assumed non-opened cyclic oxirane groups of PGMA. Secondly, by the comparison of non-treated and treated P2VP C1s spectra additional oxygen-containing carbon species were found typical for carboxylic acids or carboxylic esters. Especially after gamma-irradition of P2VP brushes the ratio of silicium was increase. For that reason a oxidative polymer degradation with different carbonyl species as intermediates are expected. Furthermore the intensity of shake-up peaks was reduced confirming the destruction of the pyridine ring. Same results were obtained by the consideration of the N1s spectra. Compared to PS the resistance of P2VP brushes to the sterilization processes is reduced. The third polymer brush PNIPAAm seems to be the stable as PS. All C1s spectra are almost identically and only a slight amount of carboxylic ester species were monitored after gamma-sterilization.

Generally, the XPS spectra in case of PAA brushes could identify a mixture of carboxylic acid- and carboxylic ester-functionalised polymer brushes. Unfortunately, under such circumstances the PtBA hydrolysis was not completed. Also for this PAA polyelectrolyte brush same as for P2VP a certain oxidative polymer degradation was observed caused by gamma-irradiation.

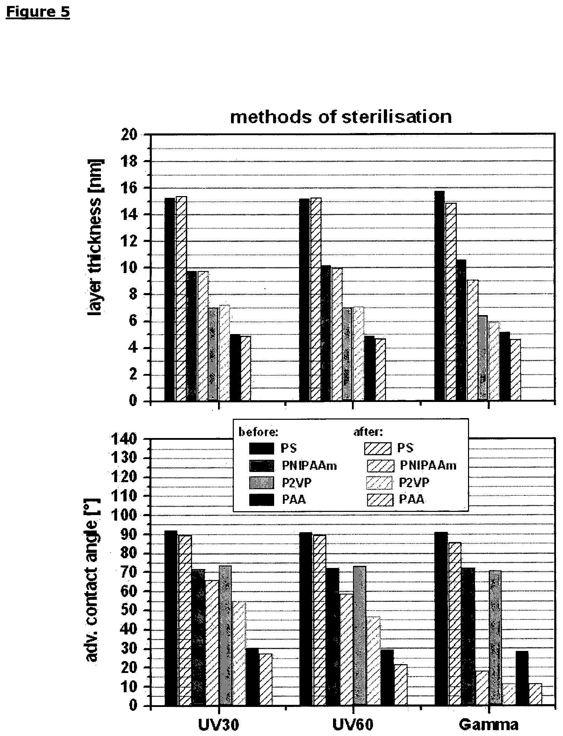

Summarising the XPS results gamma-irradiation has shown the highest damage and change of all homopolymer brush surface compositions combined with an remarkable incorporation of oxygen. In general the homopolymer brushes stability and structural integrity could be displayed in the following order: PS $\geq$ PNIPAAm > PAA > P2VP. The data shown in figure 5 are consistently with the conclusions of the XPS evaluation. The influence of sterilisation conditions on layer thickness and on water contact angle follows the same order as mentioned above for the XPS approach. While the layer thickness decreased less the water contact angles particular for the gamma-irradiated polymer brush samples were dramatically reduced compared to unmodified samples. Finally considered, these specially designed homopolymer brush surfaces are biocompatible materials and are delivered sterile by the annealing process therefore eliminating the need for cleaning and sterilization before applying to cell culture experiments.

Unspecific Protein Adsorption onto Homopolymer Brushes as Initial Process

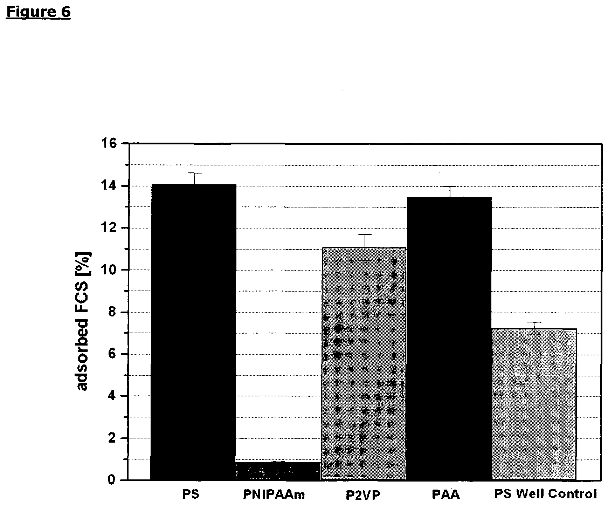

After 2 hours incubation at 37°C and under physiological conditions (pH 7.4), the supernatant was carefully obtain from the probes. The adsorption ratio was calculated from the amount of protein reduction. As the result showed in figure 6, PNIPAAm polymer brushes adsorbed only few amount of serum proteins compare to other three kinds of polymer brushes. The driving forces for adsorption on neutral hydrophobic PS bruhes are hydrohobic interaction. In case of polyelectrolyte PAA and P2VP brushes the electrostatic interaction with the serum proteins are playing mainly the role. Having investigated the protein adsorption affinity for the homopolymer brushes it is further desired to prepare polymer brushes with specific properties assigned to selected environmental conditions.

Influences of Polymer Brush Surface on Cell Behaviour

During our cell adhesion study on different polymer brush surfaces the most unique cell behaviour were found on PNIPAAm polymer brushes. From the beginning of our experiments, a variety of 3D cell spheroids on PNIPAAm polymer brush samples were obtained under our optimised cell culture conditions WITHOUT using any additional support material or equipment. According to these remarkable experimental results, in the following the possibilities and potential of THIS special 3D cell spheroid formation system will be discussed.

General Comparison of Cell Adhesion:

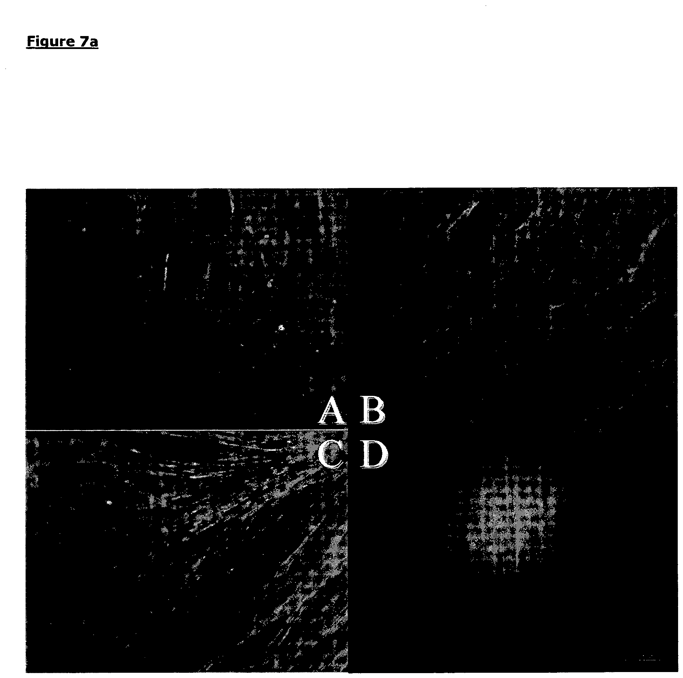

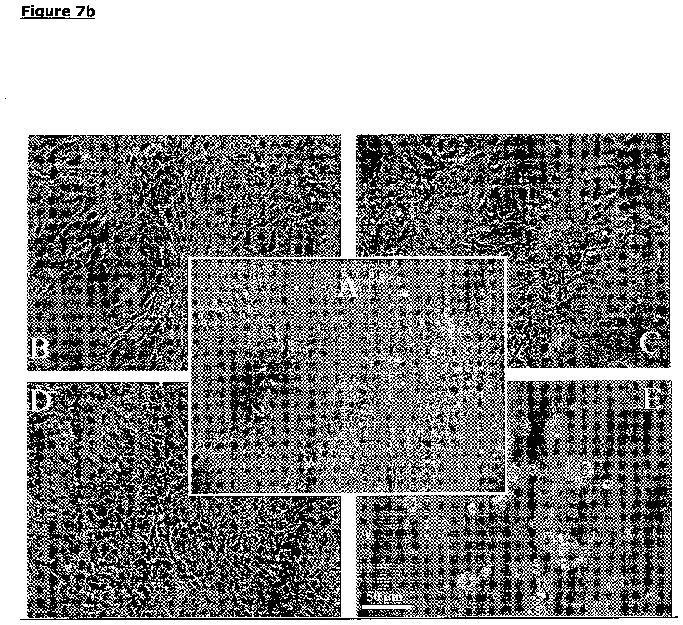

At the time point of 4 hours after seeding NHDF or hMSC cells on polymer brushes, differences between each cell morphology have been detected. All polymer brush samples (PS, P2VP, and PAA) except PNIPAAm formed 2D cell monolayers under the selected culture conditions. Certainly, P2VP and PAA homopolymer brushes had the most similar monolayer morphology compared to PS well control. However cells on PNIPAAm polymer brushes did not form a monolayer. The cells kept in round shape instead of stretching their cell bodies. After less than 24 h of incubation time, we could observe the 3D cell spheroids formation on PNIPAAm polymer brush samples. The diameter of 3D spheroids was in the range of 50-200 $\mu$m within 24 hours after seeding. Both cell types NHDF and hMSC formed 3D spheroids within 24 hours, spheroids were completely detached from the surface of PNIPAAm brushes after 72 hours, illustrated in Fig. 7a and 7b. Possible reasons for the different behaviour of PNIPAAm polymer brushes could be their unique properties. It is highly hydrophobic at 37oC and additionally, proteins were hardly able to adsorb on the surface of PNIPAAm polymer brushes in contrast to PS. The appearance of attached cells on PS homopolymer brushes as on P2VP and PAA brushes have shown similar monolayer morphology like on the PS well control but under certain circumstances (UV modification, different cell medium DMEM and RPMI-1640) the formation of 3D spheroids was also determined on PS brushes. Indeed this spheroid formation process on PS brushes was not reliable and reproducible.

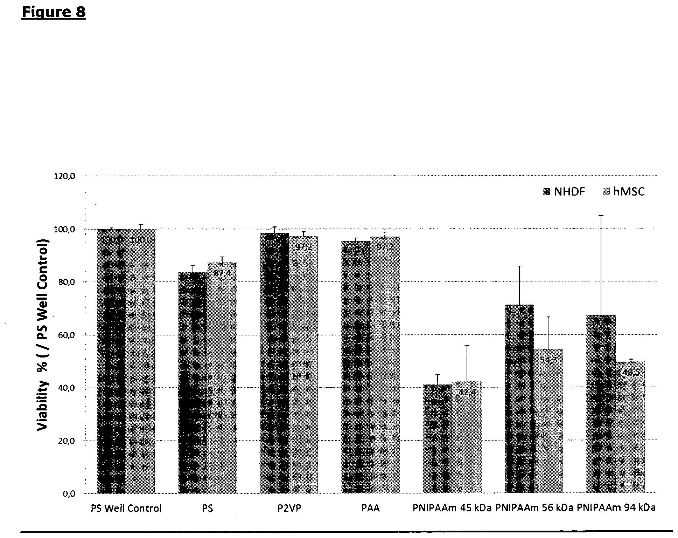

Regarding the results of the viability tests there was no significant differences between adhered NHDF and hMSC. Both cell types has been maintaining their viability after adhesion on polymer brushes almost same as on the PS well control, as one can see in Fig. 8. Considering the neutral homopolymer brushes PS and especially PNIPAAm which tends to form spheroids the value of viability was lower than the values for cell monolayer-forming brush samples. For the first moment it could cause a misunderstanding of the data but there is an experimental factor. During the viability test the diffusion speed of staining reagent into spheroids is slower than into 2D cell monolayers at the same reaction time of 3 hours. Therefore the viability data seems to be decreased. Cell re-seeding experiments and further observation by means of LSM has proven that the center of 3D cell spheroids was viable which will be discussed later on.

Influence of sterilisation on cell behaviour:

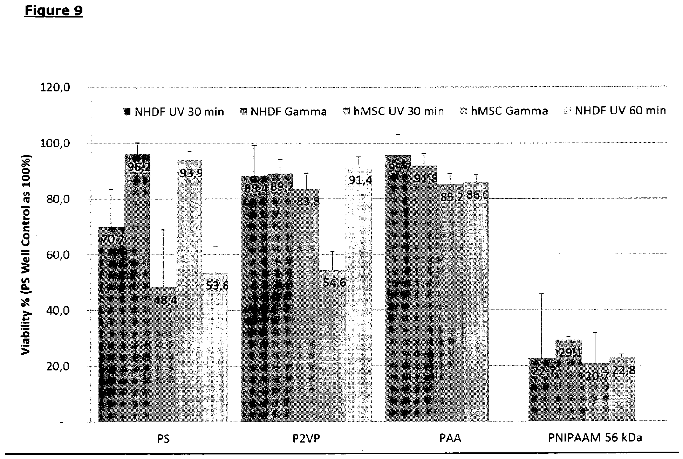

In order to determine the influences of UV- and gamma-irradiation the cell culture experiments were performed on 4 different homopolymer brushes. Figure 9 summarizes the viability analysis. In general a significant distinction could be figured out between the single homopolymer brushes itself unlike in figure 8. Taken into account the above mentioned results of the surface characterization after sterilization the change of viability data is reasonable. Particularly, the viability values of PS brushes are unsteady because on certain UV-modified PS polymer brushes the formation of 3D spheroids was occurred (images not shown).

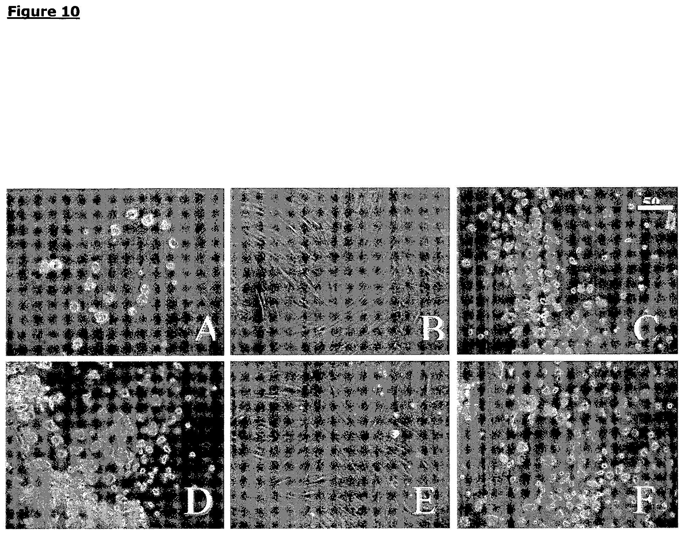

Just like in figure 8, cells on sterilized PNIPAAm polymer brushes have also formed spheroids but with totally lowered viability. Nevertheless, for example the comparison of images A (UV+) and D (UV-) in figure 10 show no differences in spheroid morphology on PNIPAAm brushes. While there was no hindrance for the 2D cell monolayer spreading on sterilised PAA brushes so that the viability was specified as for non-sterilised PAA brush samples (comparison of figure 8 and 9) the gamma-sterilised P2VP brush surface has changed especially.

The gamma-irradiated P2VP polymer brushes tend to form spheroids (images not shown) therefore the viability value is decreased according the above explained experimental reason and furthermore the cell proliferation was lesser. In summary, the physical modification of polymer brushes as it occurs by means of sterilization has a certain impact on the chemical composition of the polymer brush surfaces and consequently an influence on the cell behavior. For this reason, finally we carried out the cell culture experiments on polymer brushes without any sterilization procedure in advance and we found no alterations, as one can see in figure 10 (D and F).

Effect of Culture Conditions:

In order to investigate the dependence of the NHDF and hMSC cells on their environment several cell culture parameters were changed. Furthermore, these experiments have demonstrated that the influence of the polymer brush properties itself has to be consider together with the efficiencies of the chosen cell culture conditions. Below, the results of the 3D cell culture spheroids on different PNIPAAm polymer brushes will be discussed in detail:

3D Spheroid Cell Culture on different PNIPAAm Polymer Brushes

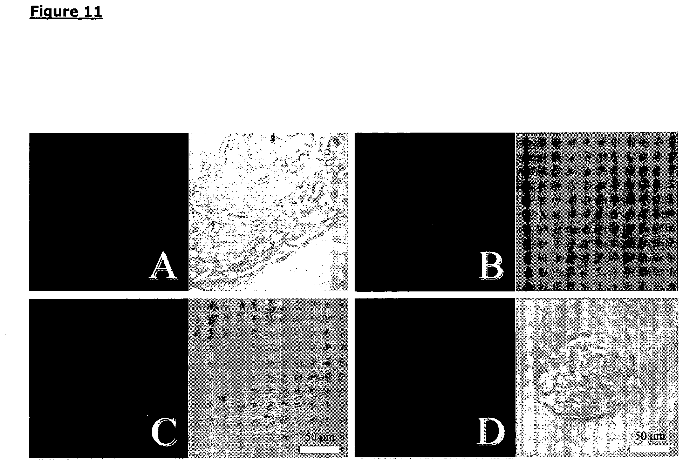

Differences between NHDF and hMSC spheroids: During the cultivation of NHDF and hMSC on diverse PNIPAAm polymer brushes certain significant differences were observed for each cell type. In general the size of spheroids varied between 50-200 $\mu$m, even some of spheroids were smaller than 50 $\mu$m. Currently, the spheroid size distribution is still wide. Almost all cell spheroids detached from the surface of PNIPAAm polymer brushes after 72 h cultivation and were floating in the cell culture reservoir. Apparently, the diameter of hMSC spheroids were slightly smaller than NHDF spheroids on PNIPAAm polymer brushes, illustrated in Fig. 11. Additionally, the hMSC spheroid cultivation was always faster than the formation of NHDF spheroids independent on the selected culture conditions.

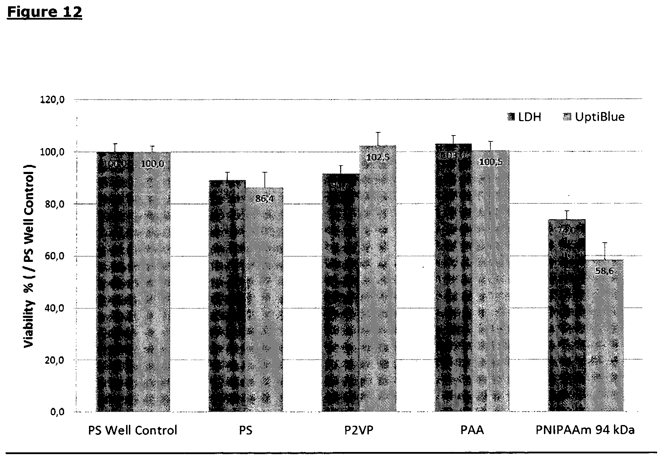

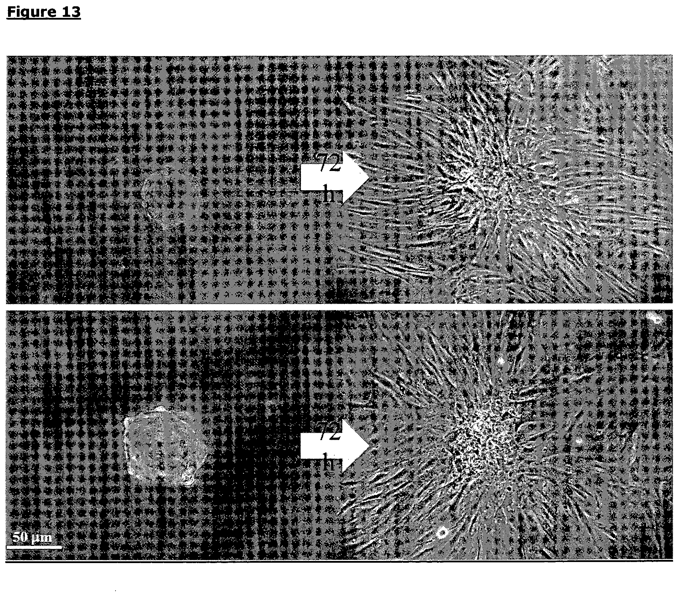

Evidence of cell viability of spheroids by re-seeding of transferred spheroids: The former described problems with the correct viability evaluation of formed spheroids were tried to be solved by using another viability test, the LDH assay. In case of 3D spheroids on PNIPAAm brush samples the viability was lowered (30% of control) because the reagent of the viability test can not defuse into the center of 3D spheroids in order to react as same as for 2D monolayers. Applying the LDH assay we found out that the viability value of spheroids were higher than in case of UptiBlue assay (60% of control). The comparison of both viability assays is shown in figure 12. Even though the LDH assay supports to overcome the difficulties with the evaluation of the cell viability inside of spheroids still the values are lower than for 2D monolayer samples. This could be reasonable because when cells formed 3D spheroid they differentiate more than they proliferate. The 3D morphology accelerates the cell-cell communication. To definitely prove the viability of the cells in 3D spheroids, we performed re-seeding experiments. Both spheroid of NHDF and hMSC were proliferated on PNIPAAm brushes, gentle harvested and finally re-seeded on PS wellplate surface which in all described cell experiments functioned as the control surface. As one can see in figure 13 cells were stretched into monolayers and behave identical as one expected on PS well control (compare with figure 7b (A)).

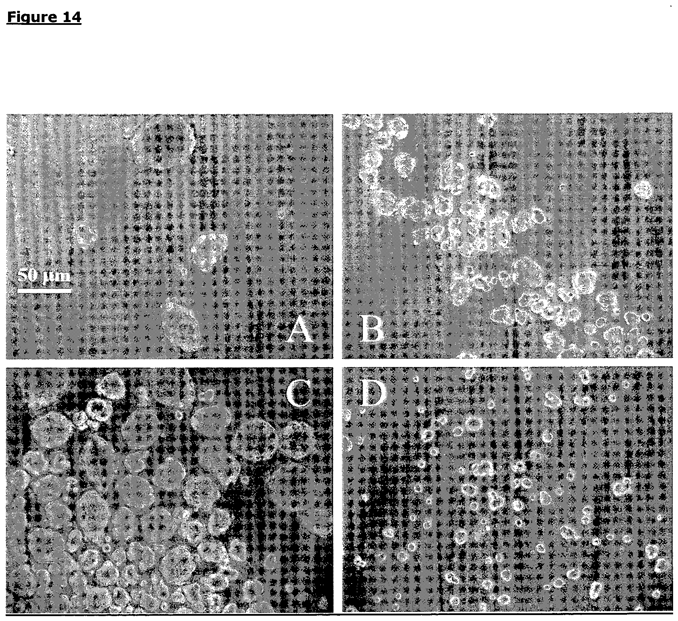

Influences of cell density and pre-treatment conditions on spheroid size and morphology: It is well-known that the density of cells influences the cell interaction. As a standard protocol, we employed $5 \times 10^4$ cells/sample from the beginning of this study. In addition, whether increasing or decreasing of NHDF or hMSC cell density (in the range from $2.5 \times 10^4$ cells/sample to $1 \times 10^5$ cells/sample) 3D spheroids were exclusively formed instead of monolayers on PNIPAAm polymer brush samples. Even the cultivation time was prolonged over 72 hours the 3D morphology did not change, dramatically. Figure 14 summarizes the NHDF adhesion results applying different NHDF cell densities on PNIPAAm polymer brushes. It is shown that the higher cell number of sample (C) caused a slightly increased number of NHDF spheroids compared to sample (A).

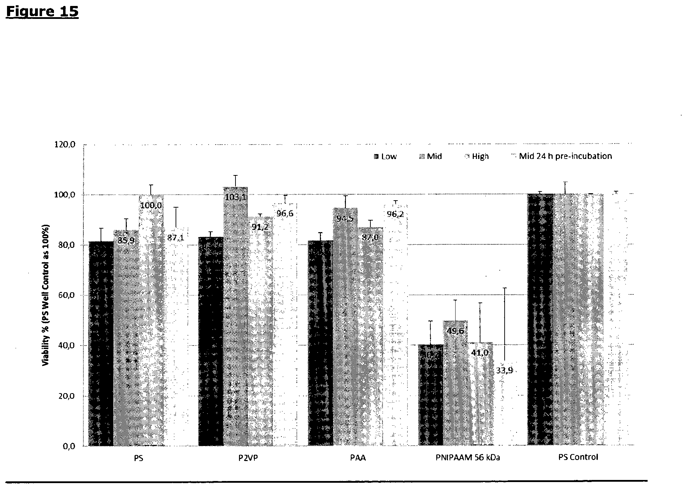

Sample (D) in Figure 14 describes a further experiment thereby the PNIPAAm brushes were additionally pre-treated using pre-warmed PBS (37°C) for 1 hour and subsequently culture medium containing 10% (v/v) FCS. The polymer brush samples were stored under medium for 2 or 24 hours in the incubator at 37°C. After 24 hours of culture medium pre-treatment, formation of spheroids were disturbed compared to 2 hours samples. This PNIPAAm polymer brush sample shows 3D NHDF spheroids with diameter smaller than for other culture conditions. That changed cell aggregation behavior could be influenced by the amount of proteins adsorbed onto the polymer brush surfaces. The variation of the cell density has no impact on the viability of the cells. In case of PNIPAAm the viability values of the cells were not influenced by the size of the spheroids (Fig. 15).

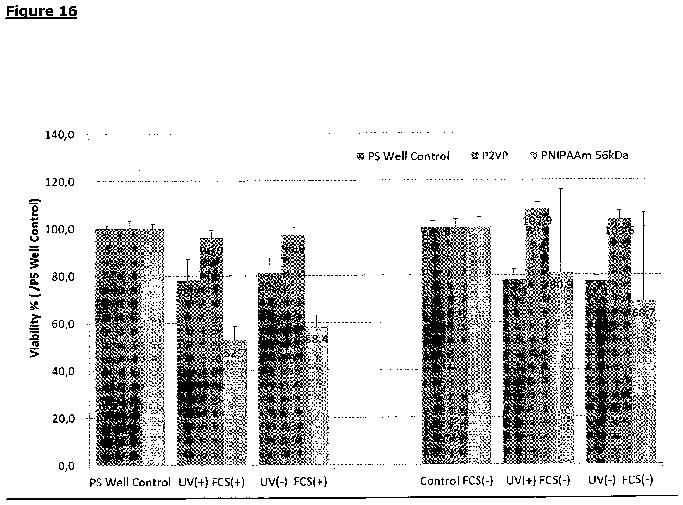

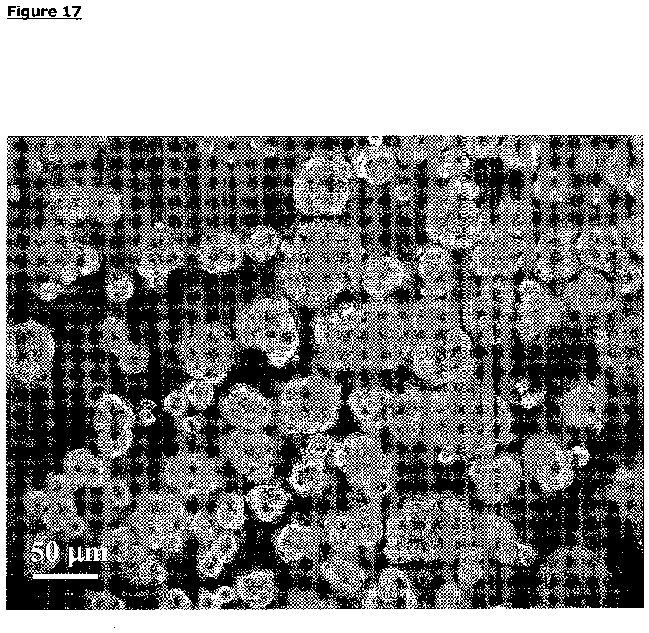

For further experiments of this study, 1 hour PBS and 2 hours culture medium pre-treatment were the selected parameters as a standard pre-treatment protocol. These results suggested the great influences of pre-adsorbed FCS proteins on polymer brushes as key component of cell attachment on surfaces. In order to determine the importance of FCS especially for the spheroid formation on PNIPAAm brushes additional approaches have been done. The cell preparation (subculture) was performed with 10% FCS in comparison with serum free medium firstly employed for 72 hours cell cultivation. The results in figure 10 and Figure 16 have shown that even without FCS, PNIPAAm polymer brush samples formed 3D spheroids certainly with smaller diameter. The viability results of these specific samples in Figure 10 have to be discussed conditionally because of the high error bars. We suppose that among with the hMSC spheroids single cells were still available after 72h adhesion. It seems the hMSC spheroid formation without FCS took place at limited rate. However, as shown in Fig. 17, continuous hMSC spheroid cultivation longer than 3 days and under serum free conditions generates considerable bigger spheroids in size. That means in general the spheroid formation process is continuously possible over several days with remarkable size increase independent on content of FCS.

Interestingly, in contrast to the former research results, when the seeded cell density was higher than $2 \times 10^5$ cells/sample we observed explicitly cell monolayers even on PNIPAAm polymer brush samples. This formed monolayer can be detached as complete cell sheet from the PNIPAAm surface by a subsequent cooling step (data not shown).

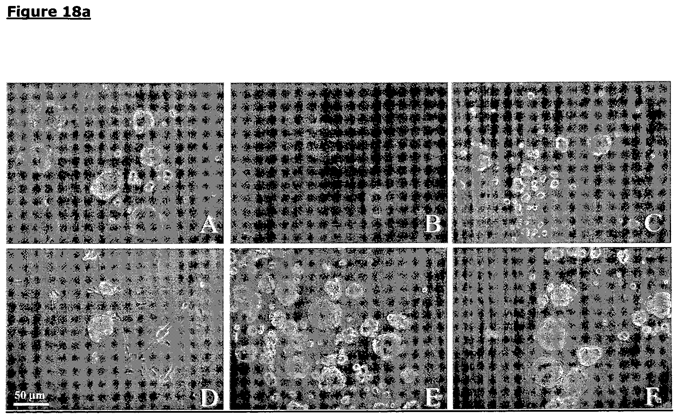

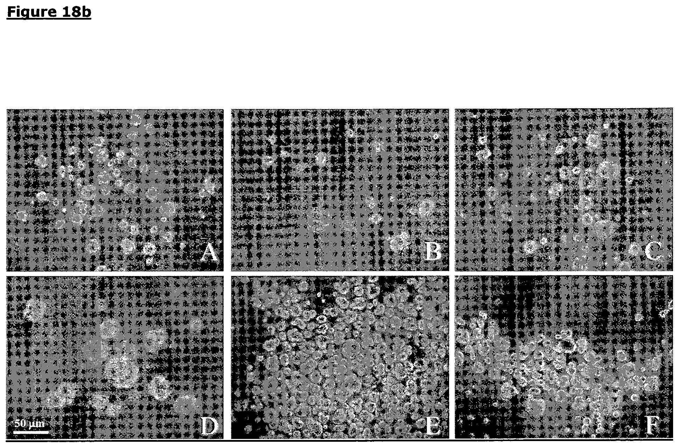

Effect of PNIPAAm molecular weight: We prepared PNIPAAm polymer brushes with PNIPAAm polymers of different molecular weights (45, 56, 94 kDa) as well as using other PNIPAAmC12 polymers containing a hydrophobic carbohydrate chain at the end (91, 135, 178 kDa). Each polymer brush length which is related to the molecular weight triggered the generation of 3D spheroids. There were no differences in the cell behavior on simple PNIPAAm brushes compared to PNIPAAmC12 brushes. Currently, a molecular weight dependence could not be find out. The images of figures 18a and 18b verify these achieved results. As specified in Figure 8 and already discussed above there is a significant size differences between NHDF spheroids and hMSC spheroids after same cultivation time.

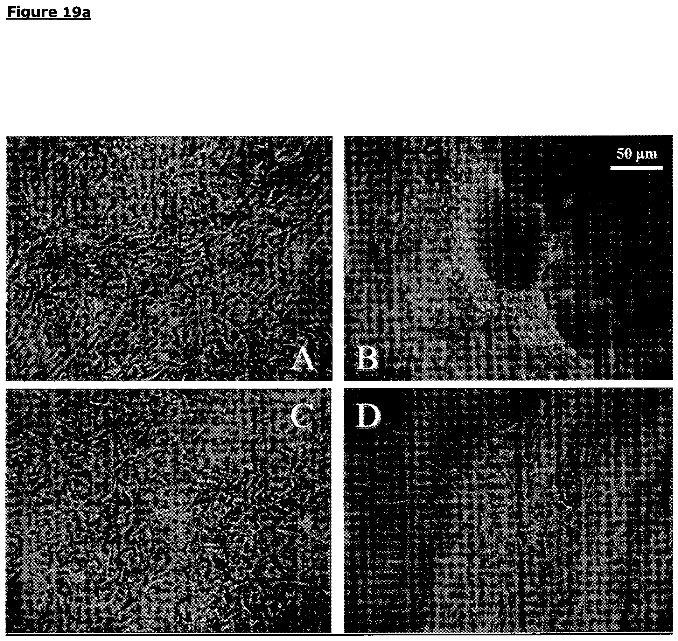

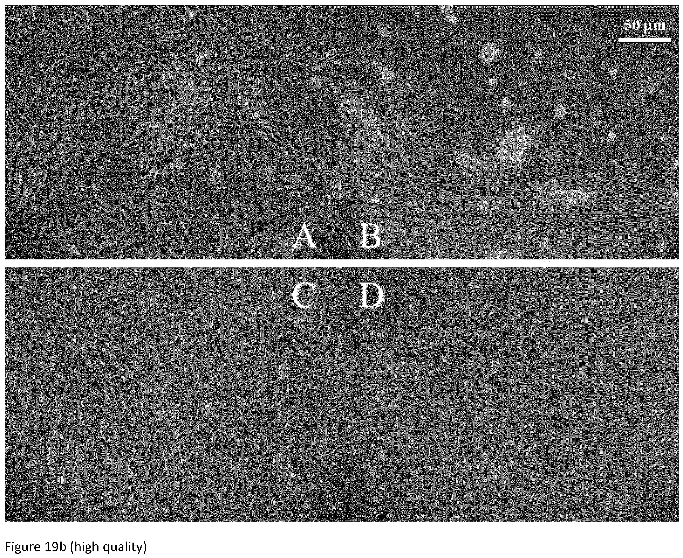

PNIPAAm containing binary brushes vs homopolymer brushes: When binary polymer brushes (PAA-PNIPAAm and PS-PNIPAAm) were applied for cell culture approaches every morphological change were being caused by the PNIPAAm component. While a lower content of PNIPAAm (20%) caused a cell monolayer the higher content of PNIPAAm (80%) showed the evidence of cell aggregation toward to spheroid formation for each NHDFs and hMSCs. Higher ratios of PAA or PS suppress the 3D cell culture spheroids. Not any of the binary brush samples formed complete 3D spheroids in the same 3D morphology as on PNIPAAm homopolymer brushes in time range of 72 hours. Except for hMSCs on PS-PNIPAAm binary polymer brushes in the ratio of 20:80 spheroids were detected acceptably (Fig. 19a and 19b). Generally, the binary polymer brush samples have shown unique characteristics in common.

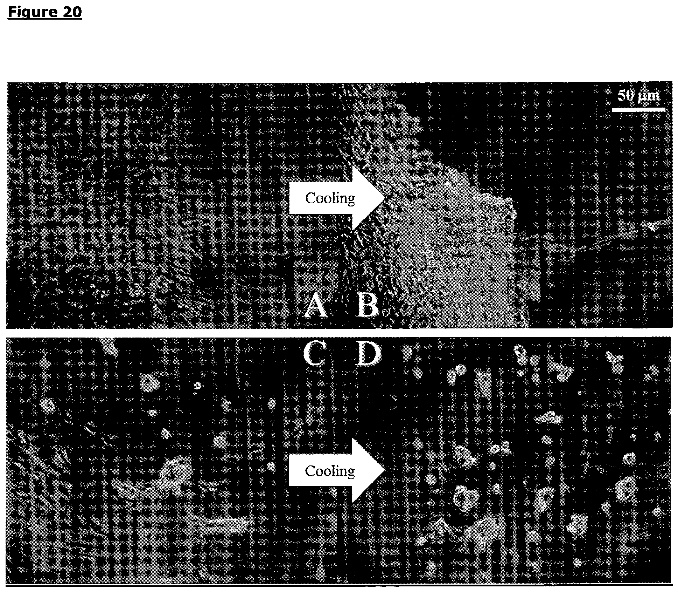

Cell behavior responses to temperature switching: PNIPAAm homopolymer brushes and binary polymer brushes containing PNIPAAm have been investigated for their stimuli responses by changing the temperature between 37°C and 4°C. When the PAA-PNIPAAm binary polymer brush sample with a ratio of 20:80 was cooled down under the condition of 4°C for 10 min the cell monolayer was detached from the sample surface. Binary polymer brushes with higher ratio of PNIPAAm (80%) combined with a PS component (20%) showed 3D spheroid morphology as same as PNIPAAm homopolymer brush samples. Observing their temperature response there was a difference in the cell spheroid behavior dependent on surrounding temperature. In case of PS-PNIPAAm binary brush with a ratio of 20:80, spheroids were not detached from the sample surfaces at 37°C, however shifting the temperature to 4°C floating spheroids were obtained (Fig. 20). This experiment proves that binary PNIPAAm brushes are temperature-responsive polymers which are reacting by temperature changes (Lower Critical Solution Temperature < 32°C). Indeed the induced reaction is on the one hand affected by the second component of the binary PNIPAAm brushes and on the other hand by the correlated proportion of each component.

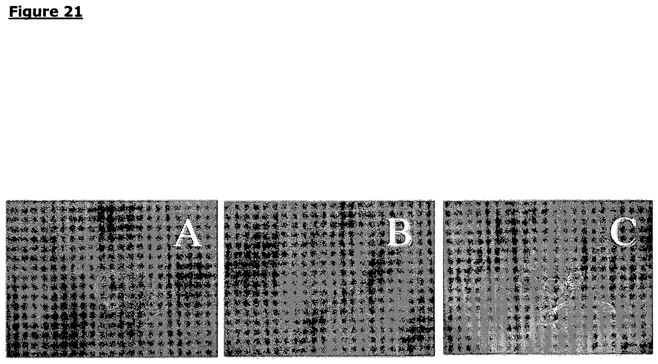

Reuse after Trypsin/EDTA Treatment: The last examinations in our study have considered practical aspects and relevance of polymer brushes in terms of durability and reusability. We could estimate that PNIPAAm polymer brush surfaces were able to reuse due to their polymer brush specificity. In the carried out experiment after trypsin/EDTA treatment, the PNIPAAm polymer brush samples were able to form 3D cell spheroids repeatedly. Shown in figure 21 it was proved that the same PNIPAAm brush sample once used for cell culture experiment could be applied again for next experiments after trypsin treatment.

Considering the impact of the sterilisation on the polymer brush stability we found out that the conditions of the polymer brush preparation process itself fabricates samples which are already in the essential sterilised quality as it is required for cell culture experiments. This conclusion increases the usability of our polymer brushes as fundament for a new 3D cell culture method.

Regarding cell culture analysis, the PNIPAAm polymer brush could be particularly the selected candidate for a new and specific 3D cell culture method. PNIPAAm polymer brushes formed 3D cell spheroids with both NHDF and hMSC within 24 hours. Generally, hMSC spheroids are always smaller than NHDF spheroids and the spheroid size distribution is still wide. In case of cultivation on PNIPAAm homopolymer brushes no significant dependence on molecular weight occurred.

There are several advantages of PNIPAAm homopolymer brush samples among the spheroids formation without any need of additional materials so that it is possible to prevent certain contamination.

Furthermore it is to highlight that PNIPAAm polymer brushes can be used repeatable by applying enzymatic treatment or temperature stimulation therefore reducing the cost performance. We strongly believe, using PNIPAAm polymer brushes has a great perspective to produce spheroids in one-step without any additional protocols and changes in current culture condition. As it was specified even by use of serum free medium spheroids were generated on PNIPAAm brush surfaces and survived longer than 3 days with reasonable viability.

This allows also 3D cell culture systems applied for cell co-culture to evaluate more complicate biological functions under similar environments. With its capability of rapidly forming spheroids the present invention can be used in regenerative medicine, tissue engineering and particularly to the stem cell field where the cellular microenvironment have a profound influence.

The following non-limiting Examples are given to further illustrate the invention. They shall not be construes to be limiting.

**Examples**

Example 1

Preparation of homopolymer brushes

In all experiments the homopolymer brushes were coated either on cover glass (VWR, Germany, diameter 15 mm) for the application in the cell culture experiments or on single-crystal silicon wafers. Highly polished single-crystal silicon wafers of {100} orientation with a native $SiO_2$ layer thickness of about 2 nm were obtained from Wacker Chemtronics (Germany) and have been used to determine the layer thicknesses by means of ellipsometry.

The following functionalized polymers were purchased from Polymer Source, Inc. (Canada): Monocarboxy-terminated poly(N-isopropylacrylamide) with various molecular weights, monocarboxy-terminated poly(2-vinylpyridine), monocarboxy-terminated poly(styrene), monocarboxy-terminated poly(t-butylacrylate) and the adhesion promoter poly(glycidyl methacrylate).

**TABLE 1:** Overview of all applied functionalised polymers for homopolymer brush and binary brush preparation; PNIPAAm[C12] samples possess more narrow weight distribution and were prepared by RAFT polymerisation; the other PNIPAAm samples were prepared by free radical polymerisation:

| POLYMER | Molecular weight Mn [g/mol] | PDI | abbreviation |
|---|---|---|---|
| Poly(glycidyl methacrylate) | 17500 | 1.70 | PGMA |
| Poly(N-isopropylacrylamide) | 45000<br>56000<br>94000 | 1.40<br>1.40<br>1.40 | PNIPAAm |

(continued)

| POLYMER | Molecular weight Mn [g/mol] | PDI | abbreviation |
|---|---|---|---|
| Poly(N-isopropylacrylamide) (C12-chain end group) | 91000<br>135000<br>178000 | 1.15<br>1.30<br>1.36 | PNIPAAM$^{C12}$ |
| Poly(2-vinylpyridine) | 40600 | 1.08 | P2VP |
| Poly(styrene) | 48000 | 1.05 | PS |
| Poly(t-butylacrylate) (after hydrolysis: polyacrylic acid PAA) | 42000 | 1.12 | PtBA |

All organic solvents were purchased from Merck KGaA (Germany), dried according to the established procedures, and freshly distilled before using.

Prior to the grafting of polymers the glass and silicon substrates have been cleaned firstly with absolute ethanol in the ultrasonic bath for 20 min and in a second step by oxygen plasma treatment (100W, 1min).

Subsequently, a 0.02 wt % solution of PGMA in chloroform was spin-coated (2000rpm, 10sec) on the substrates (Spin 150, SPS Coating, The Netherlands) and annealed under vacuum at 100°C for 20 min to crosslink PGMA, so that the silanol groups of the substrate react with a fraction of the epoxy groups of PGMA. Thus forming an anchoring layer of $2.5\pm0.2$nm thickness equipped with remaining epoxy groups for the following "grafting-to" process.

Afterwards, in order to obtain homogeneous grafted homopolymer brush layers, a 1wt% solution of the respective monocarboxy terminated polymer in THF was spin-coated onto the anchoring PGMA layer (2500rpm, 10sec). Adhesion via ester bonds was performed by annealing the coated substrate in a vacuum oven (Vacutherm®, Heraeus, Germany) overnight for 16 hours (PS and P2VP 120°C; PNIPAAm 160°C and PtBA 100°C). The annealing temperature was chosen above the glass transition temperature of the polymer. Noncovalently bound polymer was finally removed by Soxhlett extraction for 3 hours in THF.

Additionally, in case of grafted PtBA brush layer a concluding hydrolysis step (15min, 50 rpm stirring in a flask with methanesulfonic acid and dichloromethane) was necessary to achieve a poly(acrylic acid) homopolymer brush (PAA) with free carboxylic groups along the polymer chain. The final PAA homopolymer brushes were treated by extraction in ethanol, repeatedly rinsed in deionised water (Millipore, Germany) and dried under nitrogen flux. It is recommended to store all readily prepared homopolymer brushes exclusive from day light.

Example 2

Preparation of binary polymer brushes

The preparation of binary polymer brushes starts with the grafting-to procedure of the more hydrophobic polymer component, for example with polystyrene in case of the PS-PNIPAAm (45kDa) binary brush. The preparation protocol for each polymer component is similar to the process of the respective homopolymer brush. However, important is the difference of the annealing time: the PS layer tempering at 120°C for 120 min cause a ratio of 80 to 20 percentage (PS:PNIPAAm), while annealing for only 10 minutes results in the binary brush PS:PNIPAAm with a proportion of 20 to 80. The subsequent PNIPAAm brush layer was prepared following the homopolymer protocol. The preparation of the binary brush PAA-PNIPAAm is an exception. In this case, the more hydrophilic PtBA brush layer has to be coated firstly because of the included final hydrolysis step. Annealing of the PtBA layer at 100°C for 16h gives a final ratio of 80 to 20 (PAA-PNIPAAm) while annealing at 120°C for 20 min results in a ratio of 20 to 80 of the binary brush. Afterwards the second polymer component PNIPAAm will be grafted as described before.

Example 3

Characterization of the polymer brush surface

First of all the characterization of the polymer brush surfaces is required for a better understanding of the following interaction between the seeded cells and the homopolymer brushes in our research. Analytical methods applied within this work have been X-ray Photoelectron Spectroscopy (XPS), dynamic contact angle measurements and ellipsometry.

*Ellipsometry:* The thickness of the silicon oxide layer and grafted homopolymer brushes after preparation were measured using a micro-focus ellipsometer (Sentech SE-402, Sentech Instruments GmbH, Berlin, Germany). The wavelength was $\lambda$ = 633 nm with an angle of incidence of 70°. A multi-layer model for a flat film using the experimentally measured ellipsometric angles $\varphi$ and $\Delta$ was applied to calculate the thickness of the layers.

For further ellipsometric experiments the dry as well as swollen layer thicknesses and refractive indices were determined with a spectroscopic ellipsometer (M-44, J.A.Woollam Co., Inc., Lincon, NE), recording $\Delta$ and $\tan(\psi)$ at 44 wavelengths within the range of 400-800 nm. All values were estimated at a 68° angle of incidence. Swelling was performed in 0.01 M PBS buffer at pH 7.4 using a batch cuvette (TSL Spectrosil, Hellma, Muellheim, Germany). A home-built Peltier heating stage equipped with measuring software programmed with TestPoint (measurement Computing Corporation, Norton, MA) was used to estimate the swelling behavior of PNIPPAm in the temperature rate of 4° - 40° C. For this reason an extra coverage of the glass cell was used and constant nitrogen flow was applied during measurement to ensure for not freezing cell and sample at very low temperatures.

*X-ray photoelectron spectroscopy* (XPS): The analysis was carried out by means of an AXIS ULTRA photoelectron spectrometer (KRATOS ANALYTICAL, Manchester, England). The spectrometer was equipped with a monochromatic A-K$\alpha$-X-ray source of 300 W at 15 kV. The kinetic energy of the photoelectrons was determined with a hemispheric analyzer set. During all measurements electrostatic charging of the sample was overcompensated by means of a low-energy electron source working in combination with a magnetic immersion lens. All spectra were adjusted to the hydrocarbon reference peak. Quantitative elemental compositions were determined from peak areas.

*Contact Angle Measurement:* The contact angle measurements provide information concerning the surface free energetic state and wettability. All measurements were carried out as dynamic sessile drop experiments employing an OCA40-micro-goniometer (Data-Physics Instruments GmbH, Filderstadt, Germany) at relative humidity (50%), controlled room temperature (T=22°C) and additional at 40°C. The test liquid was freshly deionised water with a surface tension of 72.8 Nm. The advancing contact angle ($\theta_A$) was evaluated from dynamic dispensing/re-dispensing measurements with a volume of the sessile drop of 5$\mu$l to 10$\mu$l.

Example 4

Sterilization procedure for homopolymer brushes

Biomaterials or biomedical devices for tissue engineering or clinical applications like implantation must be either manufactured aseptically or sterilized before use. As new biomaterials for implantation including surface modification processes continue to be developed, the need to identify methods to safely sterilize the materials and maintain sterilized still remains. Sterilization is a critical step especially for surface modifications such as homopolymer brush coatings or hydrogel coatings and the effects of different methods on the integrity of the surface modification was investigated in our study.

The sterilization procedures have been used before cell culture experiments and either the effect of sterilization on the physico-chemical properties of the homopolymer brushes as well as on the cell behavior has been studied. Different sterilization methods have been compared with the goal to find the gentlest non-destructive method for the homopolymer brush films. The effectivity and stability of the homopolymer brush coatings on cover glass were studied after $\gamma$-irradiation (dose 30-35 kGy) and after UV-irradiation for 30 and 60 min under the laminar flow bench.

Example 5

Analysis of protein adsorption

The initial phenomena of protein adsorption are mainly responsible for further interplays because cells interact with surface adsorbed proteins rather than with the material surface itself. Consequently, this unspecific protein adsorption can lead to a variety of problems such as negatively affecting the biocompatibility of biomaterials. For this reason, we investigated the process of protein adsorption onto the described homopolymer brushes by means of fetal calf serum (FCS) which is used as supplement of the cell culture media.

During the initial adsorption experiment the homopolymer brush surfaces were incubated with 1%FCS in PBS for 2h at 37°C. Similar parameters have been chosen for the following cell culture experiments. The results were quantitatively evaluated by use of the colorimetric protein analysis assay Roti-Nanoquant® (Roth, Germany) which was carried out according to the supplied protocol and is easy and rapid to complete. The working solution of Roti-Nanoquant kit has been established as modification of Bradford's protein assay. By using the required amount of solution, reproducible protein amounts from 200ng onwards can be analysed in aqueous solutions. Each sample is measured at 590nm and 450nm and the linearity results from the quotient OD590/450.

Example 6

3D Cell culture experiments

Preliminary cell experiments were performed to investigate the cell repellence, cell viability and cell adhesion on the homopolymer brush coatings.

The homopolymer brush coatings were pretreated firstly with pure phosphate buffered saline (PBS, Sigma-Aldrich, Germany) for 1h and subsequently with cell culture medium containing 10% fetal calf serum for 2h at 37°C.

Subsequently, each sample was seeded with $5 \times 10^4$ cells/500 $\mu$l of Normal Human Dermal Fibroblast (NHDF, juvenile foreskin, PromoCell, Germany) and Human Mesenchymal Stem Cell (hMSC, kindly provided from Medical Clinic I, Dresden University Hospital "Carl Gustav Carus", Prof. Bornhauser, Germany) in 24-well-plates and incubated for 72 h at 37°C and 5% $CO_2$. For the cell experiments two different cell culture media DMEM (high Glucose (4.5g/l), with L-Glutamine, PAA, Austria) and RPMI-1640 (Sigma-Aldrich, USA) have been used and additionally supplemented with 10% fetal calf serum (FCS, Biochrom, Germany) and 10 U/ml penicillin and 100 $\mu$g/ml streptomycin (Biochrom, Germany).

The cell viability was evaluated by the colorimetric UptiBlue test (Interchim, France). UptiBlue solution was added to the culture medium at final concentration of 10% (v/v) and incubated for 3 h at 37°C and 5% $CO_2$. The measurement was done by Spectrophotometry (SpectraFluor Plus, TECAN, Switzerland) using 540 nm excitation and 595 nm emission filter. Polystyrene cell culture well was employed as 100% viability standard control. Furthermore, in comparison with the UptiBlue bioassay the colorimetric LDH cell cytotoxicity test has been applied, additionally (CytoScan LDH Cytotoxicity Assay, G-Biosciences). LDH assays is based on the measurement of lactate dehydrogenase (LDH) activity at 490nm absorbance and can be performed by lysis the cells in advance. LDH is a marker of remaining living cells.

The morphology of all cells was observed by phase contrast microscopy (Zeiss Axiovert 40 CFL, Germany) whereby images have been taken every 24 h as control of the cell culture and also fluorescence microscopy (Zeiss LSM510 META, Axioskop2 (FS mot), and AxioCam, Germany). Immuno-fluorescence staining was carried out by means of the dyes DAPI (Invitrogen, Germany), Phalloidin-AlexaFlour488 (Sigma-Aldrich, Germany) and Hoechst33342 (Invitrogen, Germany). According to the staining protocol, firstly the culture medium was removed from the samples in the well and washed 3 times with PBS. The cell spheroid samples were fixed by adding 3.7% formaldehyde solution for 30min at room temperature and subsequently blocked with 1%BSA/0.1%TritonX-100/PBS for 30min. After three times washing with PBS the mixed staining solution of DAPI and Phalloidin-AlexaFlour488 was applied and the samples were incubated for 60min.

**Claims**

1. Use of a 3D cell culture substrate comprising polymer brushes, wherein the polymer brushes comprise poly(N-isopropylacrylamide) polymers for the culture of 3D cell spheroids, wherein the polymer brushes comprise or consist of binary polystyrene-poly(N-isopropylacrylamide) brushes with a ratio of PNIPAAm of at least 80%, and wherein the grafting density of the polymer brushes is between 0.01 and 0.2 nm$^{-2}$.

2. Use of a 3D cell culture substrate according to claim 1, wherein the polymer brushes are prepared by a grafting to process for the culture of 3D cell spheroids.

3. Use of a 3D cell culture substrate according to claim 1 or 2, wherein the grafting density of the polymer brushes is between 0.02 and 0.15 nm$^{-2}$.

4. Use of a 3D cell culture substrate according to any of claims 1 to 3, wherein the polymer brushes have a polydispersity index (PDI) of at most 1.5, preferably at most 1.4 for the culture of 3D cell spheroids.

5. Use according to any of claims 1 to 4, wherein the cells are human cells, preferably Normal Human Dermal Fibroblasts (NHDF) or human Mesenchymal Stem Cells (hMSC) for the culture of 3D cell spheroids.

6. Use according to any of claims 1 to 5, wherein the cells are seeded in a concentration of less than $4 \times 10^5$ cells/ml.

7. Method for culturing 3D cell spheroids on a cell culture substrate comprising polymer brushes as defined in any one of claims 1 to 6.

8. Method for culturing 3D cell spheroids according to claim 7, wherein the cells are seeded in a concentration of less than $4 \times 10^5$ cells/ml.

9. Method for culturing 3D cell spheroids according to claim 7 or 8, wherein the 3D cell spheroids are released from the substrate by cooling to a temperature of under 30°C.


**Patentansprüche**

1. Verwendung eines 3D-Zellenkultursubstrats mit Polymerbürsten, wobei die Polymerbürsten Poly(N-isopropylacrylamid)-Polymere umfassen, für die Kultur von 3D-Zellensphäroiden, wobei die Polymerbürsten binäre Polystyren/Poly(N-isopropylacrylamid)-Bürsten mit einem Verhältnis von PNIPPAAm von mindestens 80 % umfassen oder daraus bestehen, und wobei die Pfropfdichte der Polymerbürsten zwischen 0,01 und 0,2 nm$^{-2}$ liegt.

2. Verwendung eines 3D-Zellenkultursubstrats gemäß Anspruch 1, wobei die Polymerbürsten durch ein Aufpfropfungsverfahren hergestellt werden, für die Kultur von 3D-Zellensphäroiden.

3. Verwendung eines 3D-Zellenkultursubstrats gemäß Anspruch 1 oder 2, wobei die Pfropfdichte der Polymerbürsten zwischen 0,02 und 0,15 nm$^{-2}$ liegt.

4. Verwendung eines 3D-Zellenkultursubstrats gemäß einem der Ansprüche 1 bis 3, wobei die Polymerbürsten einen Polydispersitätsindex (PDI) von höchstens 1,5, bevorzugt höchstens 1,4 aufweisen, für die Kultur von 3D-Zellensphäroiden.

5. Verwendung gemäß einem der Ansprüche 1 bis 4, wobei die Zellen humane Zellen, bevorzugt normale humane Hautfibroblasten (NHDF) oder humane Mesenchymstammzellen (hMSC) sind, für die Kultur von 3D-Zellensphäroiden.

6. Verwendung gemäß einem der Ansprüche 1 bis 5, wobei die Zellen in einer Konzentration von niedriger als 4x10$^5$ Zellen/ml ausgesät werden.

7. Verfahren zum Kultivieren von 3D-Zellensphäroiden auf einem Zellenkultursubstrat mit Polymerbürsten wie in einem der Ansprüche 1 bis 6 definiert.

8. Verfahren zum Kultivieren von 3D-Zellensphäroiden gemäß Anspruch 7, wobei die Zellen in einer Konzentration von niedriger als 4x10$^5$ Zellen/ml ausgesät werden.

9. Verfahren zum Kultivieren von 3D-Zellensphäroiden gemäß Anspruch 7 oder 8, wobei die 3D-Zellensphäroide von dem Substrat durch Kühlen auf eine Temperatur von unter 30°C freigesetzt werden.


**Revendications**

1. Utilisation d'un substrat de culture cellulaire en 3D comprenant des brosses de polymères, où les brosses de polymères se composent de polymères de poly(N-isopropylacrylamide) pour la culture de sphéroïdes cellulaires en 3D, où les brosses de polymères comprennent ou consistent en des brosses binaires polystyrène-poly(N-isopropylacrylamide) avec un rapport de PNIPAAm d'au moins 80%, et où la densité de greffage des brosses de polymères se situe dans l'intervalle allant de 0,01 à 0,2 nm$^{-2}$

2. Utilisation du substrat de culture cellulaire en 3D selon la revendication 1, où les brosses de polymères sont préparées par un greffage pour procéder à la culture des sphéroïdes cellulaires en 3D.

3. Utilisation du substrat de culture cellulaire en 3D selon la revendication 1 ou 2, où la densité de greffage des brosses de polymères se situe dans l'intervalle allant de 0,02 à 0,15 nm$^{-2}$

4. Utilisation du substrat de culture cellulaire en 3D selon l'une quelconque des revendications 1 à 3, où les brosses de polymères ont un indice de polydispersité (IPD) d'au plus 1,5, de préférence au plus 1,4 pour la culture de sphéroïdes cellulaires en 3D.

5. Utilisation selon l'une quelconque des revendications 1 à 4, où les cellules sont des cellules humaines, de préférence des fibroblastes dermiques humains normaux (NHDF) ou des cellules souches mésenchyateuses humaines (hMSC) pour la culture de sphéroïdes cellulaires en 3D.

6. Utilisation selon l'une quelconque des revendications 1 à 5, où les cellules sont ensemencées à une concentration inférieure à $4 \times 10^5$ cellules/ml.

7. Procédé de culture de sphéroïdes cellulaires en 3D sur un substrat de culture cellulaire comprenant des brosses de polymères telles que définies dans l'une quelconque des revendications 1 à 6.

8. Procédé de culture de sphéroïdes cellulaires en 3D selon la revendication 7, où les cellules sont ensemencées à une concentration inférieure à $4 \times 10^5$ cellules/ml.

9. Procédé de culture de sphéroïdes cellulaires en 3D selon la revendication 7 ou 8, où les sphéroïdes cellulaires en 3D sont libérés du substrat par refroidissement à une température inférieure à 30°C.

**Figure 1**

**Figure 2**

**Figure 3**

## Figure 4

LCST of PNIPAAm: Temperature dependent swelling

**Figure 5**

methods of sterilisation

## Figure 6

**Figure 7a**

**Figure 7b**

## Figure 8

## Figure 9

**Figure 10**

**Figure 11**

**Figure 12**

**Figure 13**

**Figure 14**

**Figure 15**

**Figure 16**

Figure 17

50 μm

**Figure 18a**

**Figure 18b**

**Figure 19a**

**Figure 19a**

Figure 19b (high quality)

**Figure 20**

**Figure 21**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **YOSHII Y et al.** *Biomaterials,* September 2011, vol. 32 (26), 6052-8
- **KIM TG et al.** *Adv Funct Mater.,* 2010, vol. 20 (14), 2303-9
- **BURKERT et al.** Protein Resistance of PNIPAAm Brushes: Application to Switchable Protein Adsorption. *LANGMUIR,* 02 February 2010, vol. 26 (3), 1786-1795
- **IDOTA et al.** Novel temperature-responsive polymer brushes with carbohydrate residues facilitate selective adhesion and collection of hepatocytes. *SCIENCE AND TECHNOLOGY OF ADVANCED MATERIALS,* 01 December 2012, vol. 13 (6), 64206
- **MIZUTANI et al.** Preparation of thermoresponsive polymer brush surfaces and their interaction with cells. *BIOMATERIALS,* 07 February 2008, vol. 29 (13), 2073-2081
- **QIAN YU et al.** Protein Adsorption and Cell Adhesion/Detachment Behavior on Dual-Responsive Silicon Surfaces Modified with Poly(N-isopropylacrylamide)-block-polystyrene Copolymer. *LANGMUIR,* 01 June 2010, vol. 26 (11), 8582-8588
- **MA PX.** *Adv Drug Deliv Rev.,* 14 January 2008, vol. 60 (2), 184-98
- **BURKERT S et al.** *Langmuir.,* 02 February 2010, vol. 26 (3), 1786-95
- **BITTRICH E et al.** *Langmuir.,* 21 February 2012, vol. 28 (7), 3439-48
- **NAGASE K et al.** *J R Soc Interface.,* 06 June 2009, vol. 6 (3), S293-S309